(19) Europäisches Patentamt
European Patent Office
Office européen des brevets

(11) EP 0 392 270 B2

(12) **NEUE EUROPÄISCHE PATENTSCHRIFT**

(45) Veröffentlichungstag und Bekanntmachung des Hinweises auf die Entscheidung über den Einspruch:
**07.05.1997 Patentblatt 1997/19**

(51) Int. Cl.$^6$: **C07C 19/00**, C07C 17/16

(45) Hinweis auf die Patenterteilung:
**16.06.1993 Patentblatt 1993/24**

(21) Anmeldenummer: **90105989.9**

(22) Anmeldetag: **29.03.1990**

(54) **Verfahren zur Gewinnung von C3 bis C4-Monoalkylchloriden**

Process for the recovery of C3-C4-monoalkylchlorides

Procédé pour la récupération de monochlorures d'alkyle en C3-C4

(84) Benannte Vertragsstaaten:
**AT BE CH DE DK ES FR GB IT LI NL SE**

(30) Priorität: **13.04.1989 DE 3912094**

(43) Veröffentlichungstag der Anmeldung:
**17.10.1990 Patentblatt 1990/42**

(73) Patentinhaber: **RWE-DEA Aktiengesellschaft für Mineraloel und Chemie 22204 Hamburg (DE)**

(72) Erfinder:
• **Osterburg, Günther**
  **D-4137 Rheurdt (DE)**
• **Reith, Wolfgang, Dr.**
  **D-4170 Geldern 3 (DE)**
• **Gluzek, Karl-Heinz, Dr.**
  **D-4234 Alpen (DE)**

(74) Vertreter: **Schupfner, Gerhard D.**
**Patentanwälte**
**Müller, Schupfner & Gauger**
**Postfach 17 53**
**21236 Buchholz (DE)**

(56) Entgegenhaltungen:
EP-A- 0 352 799            DD-A-  138 470
DE-A- 2 422 969           DE-A- 2 640 852
JP-A- 61 200 933

Bemerkungen:
Die Akte enthält technische Angaben, die nach dem Eingang der Anmeldung eingereicht wurden und die nicht in dieser Patentschrift enthalten sind.

EP 0 392 270 B2

**Beschreibung**

Die vorliegende Erfindung bezieht sich auf ein Verfahren zur Aufarbeitung des Reaktionsproduktes der kontinuierlichen Umsetzung von $C_3$ bis $C_4$-Monoalkanolen mit Chlorwasserstoff bei erhöhter Temperatur und ggf. in Gegenwart eines Katalysators zur Gewinnung von $C_3$ -bzw. $C_4$-Monoalkylchloriden durch Abtreiben des dampfförmigen Reaktionsproduktes, Reinigung und Abtrennung des Alkylchlorides durch Wasserwäsche, Trocknung und ggf. Destillation des rohen Alkylchlorides.

Alkylchloride können durch Umsetzung der entsprechenden Alkanole mit Chlorwasserstoff bei erhöhter Temperatur und bevorzugt in Gegenwart von Katalysatoren hergestellt werden.

In der DE-PS 24 22 969 ist ein Verfahren zur Abtrennung von $C_1$ bis $C_3$ Chloralkanen aus Gemischen beschrieben, die durch Umsetzung von $C_1$ bis $C_3$ Alkanolen mit Chlorwasserstoff unter Erhalt eines überhitzten Dampfes bestehend aus Chloralkan, den nichtumgesetzten Ausgangkomponenten Alkanol und Chlorwasserstoff sowie Wasser hergestellt worden sind. Nach dem Verfahren dieser deutschen Patentschrift wird der überhitzte Dampf in einer Destillationskolonne mit einer wäßrigen Lösung von Alkalihydroxid unter Bindung des überwiegenden Teiles des anwesenden Chlorwasserstoffes behandelt und die anfallende Lösung entfernt, der abströmende Alkylchlorid-Dampf in eine Alkali-Reinigungskolonne geleitet und dort mit Alkalihydroxid bei 10 bis 50°C unter Bindung des restlichen Chlorwasserstoffes gewaschen, die anfallende Waschlösung zur Behandlung des überhitzten Dampfes zurückgeführt, der gereinigte Alkylchlorid-Dampf in eine gekühlte Füllkörperkolonne geleitet, dort das nichtumgesetzte Alkanol verflüssigt und entfernt sowie das Alkylchlorid in an sich bekannter Weise isoliert.

Die Abtrennung des nichtumgesetzten Alkohols einerseits und des Reaktionswassers andererseits ist nur durch gleichzeitige Neutralisation des darin enthaltenen HCl mit wäßriger Alkalilauge zu erreichen. Neben dem Verlust an nicht umgesetztem HCl wird damit je nach Umsatz eine mehr oder weniger große salzhaltige Abwassermenge erhalten, die vor der Behandlung als Abwasser in einer Stripkolonne von organischer Fracht befreit werden muß.

Der Erfindung lag die Aufgabe zugrunde, ein kontinuierliches Verfahren zu entwickeln, das die Nachteile des geschilderten Standes der Technik nicht aufweist und inbesondere die Isolierung und Wiedereinsetzung von nichtumgesetztem HCl gestattet und den Anfall salzhaltiger Abwassermengen vermeidet.

Diese Aufgabe wird erfindungsgemäß durch das Verfahren des Anspruches 1 gelöst.

Nach einer bevorzugten Ausführungsform des erfindungsgemäßen Verfahrens wird das HCl-enthaltende Reaktionswasser in Chlorwasserstoff und Wasser aufgetrennt, der Chlorwasserstoff in die Synthese zurückgeführt und das Wasser zur Waschung des Alkylchlorids eingesetzt, wobei ggf. das Waschwasser zunächst auf einem pH-Wert $\lessgtr$ 8 gebracht wird.

Erfindungsgemäß erfolgt die Trennung von nichtumgesetztem Alkohol und HCl vom Reaktionswasser durch Destillation in einer Recyclekolonne. Als Kopfprodukt fallen dabei HCl und wasserhaltige Alkohole an, die - je nach Alkoholtyp - mehr oder weniger HCl und Wasser enthalten und die im Kreislauf zur Alkylchloridsynthese zurückgeführt werden. Am Kolonnensumpf der Recyclekolonne wird das Reaktionswasser, das maximal 21 % HCl enthält, abgezogen und ggf. der HCl-Erzeugung zugeführt.

Das erfindungsgemäße Verfahren unterscheidet sich gegenüber dem Stand der Technik in den folgenden wesentlichen Merkmalen:

Es wird grundsätzlich eine Kontaktierung mit Alkali bei der Aufbereitung des Reaktorausgangsproduktes und damit sowohl die Rückspaltung der Alkylchloride als auch die Verunreinigung von Salzsäure vermieden.

Die Menge des in der schweren wäßrigen Phase enthaltenen nichtumgesetzten Chlorwasserstoffs wird durch direkte Rückführung in den Prozeß verringert und auf maximal 21 % im Reaktionswasser begrenzt.

Es wird nicht mehr Salzsäure produziert als Reaktionswasser anfällt. Die Salzsäure fällt mit einer solchen Reinheit an, daß sie entweder als Verkaufsprodukt oder zur Chlorwasserstoffgewinnung verwendet werden kann.

Gewinnt man daraus Chlorwasserstoff, so kann das verbleibende Reaktionswasser zur Extraktion der im Alkylchlorid gelösten HCl- und Alkoholreste genutzt und damit der Frischwassereinsatz bzw. Abwasseranfall deutlich reduziert werden.

Das Trennverfahren berücksichtigt insgesamt die mit steigender C-Zahl zunehmenden azeotropen Beziehungen zwischen Alkylchloriden und Alkoholen sowie die Besonderheiten der Selektivität bei den verschiedenen Alkylchloridsynthesen.

Das nach dem erfindungsgemäßen Verfahren aufzuarbeitende Reaktionsprodukt wird durch Umsetzung des entsprechenden $C_3$ bzw. $C_4$-Monoalkanols mit Chlorwasserstoff in Gegenwart eines üblichen Katalysators, z.B. Zinkchlorid, bei erhöhter Temperatur hergestellt. Die Reaktionstemperatur bei Atmosphärendruck beträgt in der Regel etwa 100 bis 180°C, vorzugsweise 100 bis 150°C. Es wird dabei ein rohes gasförmiges Reaktionsprodukt erhalten, das neben nichtumgesetztem Chlorwasserstoff und nichtumgesetztem Alkohol Alkylchlorid sowie Wasser, die entsprechenden Dialkylether und Olefine enthält.

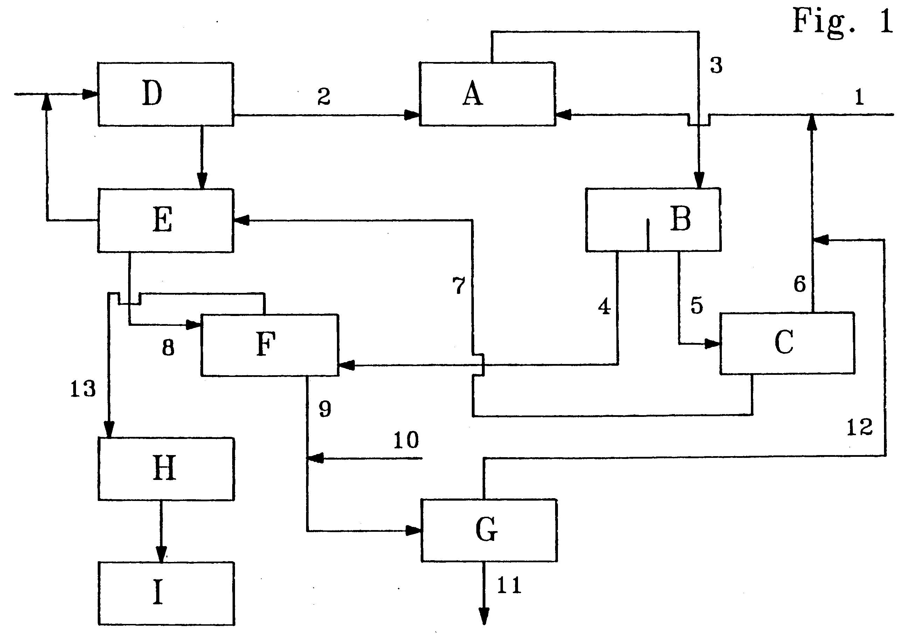

Ein Ausführungsbeispiel des erfindungsgemäßen Verfahrens ist in dem als Fig. 1 wiedergegebenen Verfahrensschema dargestellt und wird im folgenden näher beschrieben:

Frischalkohol wird über Leitung 1, ggf. nach Durchlaufen eines nicht dargestellten Abgaswäschers, gemeinsam mit

2

dem Alkoholrückführstrom über Leitung 6 und dem Chlorwasserstoff über Leitung 2 im Molverhältnis 1:1 von unten dem als Aufkocher konstruierten Reaktor A zugeführt.

Als Katalysatorlösung wird eine 60- bis 70-%ige Lösung von Zinkchlorid in Wasser vorgegeben. Hierbei richtet sich die eingesetzte Katalysatormenge nach den jeweiligen Synthesebedürfnissen ent-sprechend der vorgesehenen maximalen Katalysatorbelastung.

Die Reaktionstemperatur im Reaktor A wird den Umsatz- und Selektivitätsforderungen des jeweils zu erzeugenden Alkylchlorids entsprechend eingestellt. Das Reaktionsprodukt aus Alkylchlorid, Wasser, Nebenprodukten sowie nichtumgesetztem Alkohol und HCl wird dampfförmig aus dem Reaktor über Leitung 3 abgeführt, kondensiert und in Abscheider B in eine leichte organische Phase, die das Alkylchlorid enthält, und eine schwere Wasserphase getrennt.

Die schwere Wasserphase wird über Leitung 5 direkt der Recyclekolonne C zugeführt, in der am Kolonnensumpf das Reaktionswasser in der Regel als Maximumazeotrop von Wasser/HCl mit 21 Gew.% HCl über Leitung 7 abgezogen wird. Der im Reaktionswasser enthaltene Chlorwasserstoff wird über die zweite Stufe E der HCl-Gewinnungsanlage D/E wieder zum Syntheseeinsatz zurückgegeben. Alle anderen in der schweren Wasserphase enthaltenen Bestandteile wie Alkohol, Alkylchlorid, Azeotropwasser und der Rest an nicht umgesetztem Chlorwasserstoff werden als Kopfprodukt abgetrennt und als azeotroper Alkoholrückführstrom über Leitung 6 zusammen mit dem Frischalkohol wieder zur Synthese eingesetzt. Leichte HCl-Ausgasungen aus dem Rücklaufbehälter der Recyclekolonne C werden gemeinsam mit möglichem Abgas aus dem Abscheider B der Alkylchlorid-Synthese im Abgaswäscher (nicht gezeigt) vom Frischalkohol aufgenommen und so wieder zur Synthese zurückgeführt.

Das rohe Alkylchlorid aus dem Abscheider B, das kleine Mengen des nichtumgesetzten Alkohols, Chlorwasserstoffs, Wasser und die Nebenprodukte der jeweiligen Synthese enthält, wird unverändert über Leitung 4 dem mehrstufigen Gegenstromextraktor F zugeführt und mit Wasser gewaschen. Als Extraktionswasser kann Kondensat, zweckmäßigerweise unter Einbeziehung des neutralisierten Abwassers aus der HCl-Gewinnungsanlage D/E über Leitung 8, eingesetzt werden. Der pH-Wert eines solchen Wassers sollte nach Möglichkeit pH = 8 nicht übersteigen.

Die Extraktphase, das Waschwasser, wird anschließend über Leitung 9 abgeführt und mit NaOH aus Leitung 10 neutralisiert und in der Stripkolonne G von gelösten organischen Anteilen, wie Alkohol und Alkylchlorid, befreit. Die organischen Anteile werden über Leitung 12 dem azeotropen Alkoholrecycle von der Recyclekolonne C in Leitung 6 zugeführt und mit dem Frischalkohol zur Synthese eingesetzt. Das neutralisierte und von gelösten organischen Anteilen befreite Waschwasser wird über Leitung 11 dem Abwasser zugeführt.

Durch das Verhältnis Waschwasser : Alkylchlorid kann entsprechend den jeweiligen Verteilungskoeffizienten der für das reine Alkylchlorid spezifizierte Alkoholrestgehalt eingestellt werden.

Vor der Destillation des gewaschenen, noch feuchten Alkylchlorids, das über Leitung 13 aus dem Gegenstromextraktor F abgeführt wird, mag eine Trocknung in einem nicht dargestellten Absorber vorgesehen sein. Erweist sich diese, einer möglichen Korrosion der ersten Destillationskolonne vorbeugende Trocknung des Alkylchlorids als nicht notwendig, so kann diese entfallen. Stattdessen kann das Wasser in der ersten Destillationskolonne H azeotrop abgetrennt werden.

Die Reindestillation des erzeugten Alkylchlorids wird je nach Beschaffenheit des Produkts in unterschiedlicher Weise und abhängig von ihrem jeweiligen Nebenproduktgehalt in zwei kontinuierlich zu betreibenden Destillationskolonnen, Leichtsieder-Kolonne H und Schwersieder-Kolonne I, in an sich bekannter Weise durchgeführt.

Die folgenden Beispiele erläutern das erfindungsgemäße Verfahren. Die Ergebnisse der Beispiele sind in Tabelle I zusammengestellt.

Beispiel 1

In einem Glasreaktor mit einer lichten Weite von 40 mm und einer Länge von 550 mm, der bis zu einer Höhe von 280 mm mit einem mit Heißöl beheizten Heizmantel umgeben war, wurden 1,0 Mol = 136,3 g Zinkchlorid (70 % in Wasser gelöst) als Katalysatorlösung gegeben. Der Heißölumlauf durch den Heizmantel wurde so eingestellt, daß sich eine konstante Temperatur von 127°C in der Katalysatorlösung einstellte. Es wurden sodann stündlich 3 Mol = 220 g n-Butylalkohol und 3,4 Mol = 124 g Chlorwasserstoff gemeinsam mit 318 g eines azeotropen Rückalkohols aus der Recyclekolonne von unten zugeführt und gleichzeitig am Kopf des Reaktors 649 g Reaktionsprodukt dampfförmig abgezogen und kondensiert. Das Reaktionsprodukt wurde abgeführt und in dem Abscheider in 267 g einer leichten organischen Phase und 382 g einer schweren wäßrigen Phase aufgetrennt und separat weitergeleitet.

Die schwere wäßrige Phase wurde direkt und kontinuierlich der Recyclekolonne zugeführt, in der durch Destillation bei einer Kopftemperatur von 98,5°C und einer Sumpftemperatur von 110°C die wäßrige Phase so aufgetrennt wurde, daß am Kolonnenkopf 318 g azeotroper Alkoholrückführstrom, der unmittelbar dem Reaktor zugeführt wurde, und am Kolonnensumpf 64 g 21-%ige Salzsäure anfielen.

Bis auf den im Sumpfprodukt der Recyclekolonne enthaltenen Chlorwasserstoff, der in HCl-Aufkonzentrierungskolonnen zurückgewonnen werden kann, wurde der gesamte, bei der Synthese notwendige HCl-Überschuß gemeinsam mit dem nichtumgesetzten Alkohol und in Gegenwart von Wasser in die Synthese zurückgeführt.

Die leichte organische Phase wurde kontinuierlich in den Gegenstromextraktor geführt und mit Wasser behandelt, wobei alkohol- und salzfreies rohes Alkylchlorid erhalten wurde, das anschließend einer Destillation in den Leicht- und Schwersiederkolonnen unterworfen wurde.

In der Stripkolonne wurde dann nur noch das Waschwasser des Gegenstromextraktors, das mit Natronlauge neutralisiert wurde, abgestreift.

Beispiel 2

Gemäß Beispiel 1 wurden stündlich 4 Mol = 295 g n-Butylalkohol und 4,5 Mol = 165 g Chlorwasserstoff gemeinsam mit 549 g eines azeotropen Alkohlrückführstroms eingesetzt und bei einer Reaktionstemperatur von 127°C 993 g Reaktionsprodukt dampfförmig abgezogen und kondensiert. Durch Phasentrennung fielen im Abscheider 359 g leichte organische Phase und 634 g schwere wäßrige Phase an. Die wäßrige Phase wurde in der Recyclekolonne in 549 g azeotropen Alkoholrückführstrom und 85 g 21-%iger Salzsäure aufgetrennt.

Beispiel 3

Gemäß Beispiel 1 wurden stündlich 4,5 Mol = 333 g n-Butylalkohol und 5,1 Mol = 186 g Chlorwasserstoff gemeinsam mit 466 g eines azeotropen Alkoholrückführstroms eingesetzt und bei einer Reaktionstemperatur von 130°C 968 g Reaktionsprodukt dampfförmig abgezogen und kondensiert. Durch Phasentrennung fielen im Abscheider 405 g leichte organische Phase und 563 g schwere wäßrige Phase an. Die schwere wäßrige Phase wurde in der Recyclekolonne in 466 g azeotropen Alkohol rückführstrom und 97 g 21-%iger Salzsäure aufgetrennt.

Beispiel 4

Gemäß Beispiel 1 wurden stündlich 5,5 Mol = 408 g n-Butylalkohol und 6,2 Mol = 227 g Chlorwasserstoff gemeinsam mit 558 g eines azeotropen Alkoholrückführstroms eingesetzt und bei einer Reaktionstemperatur von 133°C 1172 g Reaktionsprodukt dampfförmig abgezogen und kondensiert. Durch Phasentrennung fielen im Abscheider 495 g leichte organische Phase und 677 g schwere wäßrige Phase an. Die wäßrige Phase wurde in der Recyclekolonne in 558 g azeotropen Alkoholrückführstrom und 119 g 21-%ige Salzsäure aufgetrennt.

Beispiel 5

Gemäß Beispiel 1 wurden stündlich 10 Mol = 739 g s-Butylalkohol und 10 Mol = 365 g Chlorwasserstoff gemeinsam mit 91,4 g eines azeotropen Alkoholrückführstroms eingesetzt und bei einer Reaktionstemperatur von 120°C 1200 g Reaktionsprodukt dampfförmig abgezogen und kondensiert. Durch Phasentrennung fielen im Abscheider 886 g leichte organische Phase und 314 g schwere wäßrige Phase an. Die schwere wäßrige Phase wurde in der Recyclekolonne in 91,4 g azeotropen Alkoholrückführstrom und 222 g 21-%iger Salzsäure aufgetrennt.

Beispiel 6

Gemäß Beispiel 1 wurden stündlich 15,0 Mol = 900 g Isopropylalkohol und 16,0 Mol = 584 g Chlorwasserstoff gemeinsam mit 62 g eines azeotropen Alkoholrückführstroms eingesetzt und bei einer Reaktionstemperatur von 118°C 1546 g Reaktionsprodukt dampfförmig abgezogen und kondensiert. Durch Phasentrennung fielen im Abscheider 1170 g leichte organische Phase und 376 g schwere wäßrige Phase an. Die schwere wäßrige Phase wurde in der Recyclekolonne in 62 g azeotropen Alkoholrückführstroms und 314 g Salzsäure mit 15,6 % HCl aufgetrennt.

Die Reaktivität in IPA bedingt nur einen geringen HCl-Überschuß bei gutem Umsatz. Aus diesem Grund ist bei diesem Beispiel der Alkoholrückführstrom HCl-frei und die HCl-Konzentration im Reaktionswasserauslaß kleiner als 21 %.

| Beispiel | | 1 | 2 | 3 | 4 | 5 | 6 |
|---|---|---|---|---|---|---|---|
| Temperatur | (°C) | 127 | 127 | 130 | 133 | 120 | 118 |
| Reaktoreinsatz | | | | | | | |
| NBA | (g/h) | 220 | 295 | 333 | 408 | | |
| SBA | (g/h) | | | | | 739 | |
| IPA | (g/h) | | | | | | 900 |
| HCl | (g/h) | 124 | 165 | 186 | 227 | 365 | 584 |
| Reaktoraustrag wäßr. org. Phase | | 1,4 | 1,8 | 1,4 | 1,4 | 0,4 | 0,3 |
| org. Phase | (g/h) | 267 | 359 | 405 | 495 | 886 | 1170 |
| Olefine | (Gew.%) | 0,7 | 0,6 | 0,8 | 1,2 | 5,9 | 0,4 |
| IPC | (Gew.%) | | | | | | 97,9 |
| SBC | (Gew.%) | 12,5 | 8,3 | 12,3 | 14,9 | 92,6 | |
| NBC | (Gew.%) | 80,7 | 82,5 | 79,7 | 76,8 | | |
| Ether + Dimere | (Gew.%) | 4,6 | 6,3 | 5,4 | 4,9 | 0,1 | 0,2 |
| NBA | (Gew.%) | 1,0 | 1,3 | 1,1 | 1,2 | | |
| SBA | (Gew.%) | | | | | 1,4 | |
| IPA | (Gew.%) | | | | | | 1,3 |
| HCl | (Gew.%) | 0,5 | 1,0 | 0,7 | 1,0 | . | 0,2 |
| wäßr. Phase | (g/h) | 382 | 634 | 563 | 677 | 314 | 376 |
| SBC | (Gew.%) | 0,8 | 0,7 | 0,7 | 1,0 | 0,7 | |
| NBC | (Gew.%) | 4,7 | 5,8 | 5,7 | 4,4 | | |
| Ether + Dimere | (Gew.%) | 1,2 | 1,7 | 1,7 | 1,3 | | |
| NBA | (Gew.%) | 35,0 | 34,7 | 34,2 | 33,0 | | |
| SBA | (Gew.%) | | | | | 18,2 | |
| IPA | (Gew.%) | | | | | | 14,4 |
| Wasser | (Gew.%) | 31,3 | 30,7 | 31,4 | 32,5 | 56,2 | 72,6 |
| HCl | (Gew.%) | 27,0 | 26,4 | 26,3 | 27,8 | 24,9 | 13,0 |
| Recycle zum Reaktor | (g/h) | 318 | 549 | 466 | 558 | 91 | 62 |
| SBC | (Gew.%) | 0,9 | 0,8 | 0,8 | 1,2 | 33,9 | |
| NBC | (Gew.%) | 8,6 | 8,7 | 8,7 | 7,3 | | |
| Ether + Dimere | (Gew.%) | 1,4 | 2,1 | 2,2 | 1,6 | | |
| NBA | (Gew.%) | 41,3 | 39,7 | 39,8 | 39,3 | | |
| SBA | (Gew.%) | | | | | 37,2 | |
| IPA | (Gew.%) | | | | | | 87,1 |
| Wasser | (Gew.%) | 22,9 | 22,6 | 22,5 | 23,1 | 6,1 | 12,9 |
| HCl | (Gew.%) | 24,9 | 26,1 | 26,0 | 27,5 | 22,8 | |
| Alkohol-Umsatz | (%) | 98,3 | 98,3 | 98,6 | 98,0 | 98,3 | 98,3 |
| Selektivität | | | | | | | |
| IPC | (%) | | | | | | 99,0 |
| SBC | (%) | 12,3 | 8,2 | 12,1 | 14,1 | 90,4 | |
| NBC | (%) | 79,8 | 81,7 | 78,9 | 76,1 | | |
| Katalysator-leistung | (mol/mol*h) | 2,3 | 3,2 | 3,5 | 4,1 | 8,9 | 14,6 |

**Patentansprüche**

1. Verfahren zur Aufarbeitung des Reaktionsproduktes der kontinuierlichen Umsetzung von C3- bis C4- Monoalkanolen mit Chlorwasserstoff bei erhöhter Temperatur und in Gegenwart eines Katalysators zur Gewinnung von C3-bzw. C4- Monoalkylchloriden durch Abtrieben des dampfförmigen Reaktionsproduktes, Reinigung und Abtrennung des Alkylchlorides durch Wasserwäsche, Trocknung und ggf. Destillation des rohen Alkylchlorides, **dadurch gekennzeichnet,** daß man

   - das durch Umsetzung bei einer Temperatur im Bereich von 100-180° C entsprechend den Umsatz- und Selektivitätsforderungen des jeweils zu erzeugenden Alkylchlorids erhaltene Reaktionsprodukt ohne destillative Trennung dampfförmig austreibt, wobei der Reaktoraustrag Anteile nicht umgesetzten Alkohols und Chlorwasserstoffs nebst Wasser enthält,
   - diesen Reaktoraustrag kondensiert, und das erhaltene Kondensat in eine leichte organische Phase und in eine schwere wäßrige Phase auftrennt,
   - die schwere wäßrige Phase unmittelbar destilliert, als Sumpfprodukt das Reaktionswasser mit maximal 21% HCl und als Kopfprodukt einen HCl- und wasserhaltigen Alkoholstrom gewinnt, den Alkoholstrom zusammen mit dem Frischalkohol in die Synthese zurückführt und
   - die leichte organische Phase mit Wasser wäscht, das Alkylchlorid abtrennt und in an sich bekannter Weise reinigt, das Waschwasser neutralisiert, zur Befreiung von gelösten organischen Anteilen abstreift und aus dem System entfernt.

2. Verfahren nach Anspruch 1, **dadurch gekennzeichnet,** daß man das HCl-enthaltende Reaktionswasser in Chlorwasserstoff und Wasser auftrennt, den Chlorwassserstoff in die Synthese zurückführt und das Wasser zur Waschung des Alkylchlorids einsetzt.

**Claims**

1. A process for working up the reaction product from the continuous reaction of $C_3$ - $C_4$ monoalkanols with hydrogen chloride at elevated temperature and in the presence of a catalyst for producing $C_3$ and $C_4$ respectively monoalkylchlorides, by stripping the vapourous reaction product, purifying and separating the alkyl chloride by washing with water; drying and, optionally, distilling the crude alkyl chloride, characterized in that

   - the reaction product obtained by reaction at a temperature ranging from 100 - 180 °C, depending on the conversion and selectivity requirements for the alkyl chloride to be produced is stripped as a vapourous product without separation by distillation, said reactor effluent containing portions of unreacted alcohol and hydrogen chloride plus water,
   - said reactor effluent is condensed and the condensate obtained is split into a light organic phase and a heavy aqueous phase,
   - the heavy aqueous phase is immediately distilled, the reaction water containing not more than 21 % HCl is recovered at the bottom of the column and an alcohol stream containing HCl and water is recovered overhead, the alcohol stream is returned together with fresh alcohol to the synthesis step, and
   - the light organic phase is washed with water, the alkyl chloride is separated and purified by prior art techniques, the water resulting from said washing procedure is neutralised, is stripped in order to remove any dissolved organic constituents, and is eliminated from the system.

2. A process according to claim 1 wherein the Hcl-containing water of the reaction is split into hydrogen chloride and water, the hydrogen chloride is recycled to the synthesis step, and the water is used for washing the alkyl chloride.

**Revendications**

1. Procédé de traitement du produit de la réaction continue de mono-alcanols en $C_3$-$C_4$ avec le chlorure d'hydrogène à température élevée et en présence d'un catalyseur pour l'obtention de monochlorures d'alkyle en $C_3$ ou $C_4$ par rectification du produit réactionnel sous forme de vapeur, purification et séparation du chlorure d'alkyle par lavage à l'eau, séchage et, le cas échéant, distillation du chlorure d'alkyle brut, caractérisé en ce que :

   - on fait sortir sous forme de vapeur, sans fractionnement par distillation, le produit de réaction obtenu par réaction à une température dans la plage de 100 à 180°C conformément aux exigences en matière de degré de transformation et de sélectivité du chlorure d'alkyle concerné devant être produit, la matière déchargée du réacteur contenant, en plus d'eau, des proportions d'alcool et de chlorure d'hydrogène n'ayant pas réagi,

- on condense cette matière déchargée, et on fractionne le condensat obtenu en une phase organique légère et une phase aqueuse lourde,
- on distille directement la phase aqueuse lourde, on recueille comme produit de queue l'eau de réaction avec au maximum 21 % de HCl et comme produit de tête un courant d'alcools contenant du HCl et de l'eau, on recycle le courant d'alcools dans la synthèse conjointement avec de l'alcool frais, et
- on lave à l'eau la phase organique légère, on sépare le chlorure d'alkyle et on le purifie d'une manière connue, on neutralise l'eau de lavage, on la rectifie pour la débarrasser des constituants organiques dissous et on la retire du procédé.

2. Procédé suivant la revendication 1, caractérisé en ce qu'on fractionne l'eau de réaction contenant du HCl en chlorure d'hydrogène et eau, on recycle le chlorure d'hydrogène dans la synthèse et on utilise l'eau pour le lavage du chlorure d'alkyle.

Fig. 1

EP 0 392 270 B2